# EUROPÄISCHE PATENTSCHRIFT

(11) **EP 4 251 987 B1**
(45) Veröffentlichungstag und Bekanntmachung des Hinweises auf die Patenterteilung: **06.08.2025**
(21) Anmeldenummer: 21809957.0
(22) Anmeldetag: 08.11.2021
(51) Int. Cl.: G01N 27/407, G01N 33/00, G01N 27/417

(54) **VERFAHREN ZUM ERMITTELN EINES FEHLERS EINES ABGASSENSORS UND ABGASSENSOR**
METHOD FOR ASCERTAINING A FAULT OF AN EXHAUST GAS SENSOR, AND EXHAUST GAS SENSOR
PROCÉDÉ DE DÉTERMINATION D'UNE DÉFAILLANCE D'UN CAPTEUR DE GAZ ET CAPTEUR DE GAZ

(30) Priorität: 24.11.2020 DE 102020214708
(43) Veröffentlichungstag der Anmeldung: 04.10.2023
(73) Patentinhaber: Schaeffler Technologies AG & Co. KG, 91074 Herzogenaurach (DE)
(72) Erfinder: WALDE, Tim, 80687 München (DE)
(74) Vertreter: Vitesco Technologies
(86) Internationale Anmeldenummer: PCT/EP2021/080874
(87) Internationale Veröffentlichungsnummer: WO 2022/111976

(56) Entgegenhaltungen:
- DE-A1- 102019 004 190
- DE-A1- 102019 203 704
- DE-B3- 102019 203 707
- DE-B4- 19 982 982

## Beschreibung

Die vorliegende Erfindung betrifft ein Verfahren zum Ermitteln eines Fehlers eines Abgassensors, beispielsweise eines Stickoxidsensors mit erweiterter Ammoniakmessung, und einen Abgassensor, insbesondere ein Verfahren zur Eigendiagnose des Abgassensors.

Abgassensoren, wie beispielsweise Stickoxidsensoren, Lambdasonden und Sauerstoffsensoren, können dem amperometrischen Messprinzip beruhen, d. h. auf einer elektrochemischen Methode zur quantitativen Bestimmung von chemischen Stoffen. Insbesondere wird an einer Elektrode des Abgassensors ein elektrischer Strom derart eingestellt, dass sich ein zeitlich konstantes elektrochemisches Potential einstellt. Beispielsweise erlauben Stickoxidsensoren eine Messung der Stickoxidkonzentration im Abgas von Brennkraftmaschinen, beispielsweise Otto- oder Dieselmotoren. Dadurch wird z. B. eine optimale Steuerung und Regelung sowie Diagnose von Stickoxidkatalysatoren durch die Motorsteuerung ermöglicht.

Derartige Abgassensoren weisen einen aus einem Feststoffelektrolyten gebildeten Hauptkörper auf, in dem Kavitäten mit zugeordneten Elektroden vorgesehen sind. Zudem ist im Hauptkörper eine Heizvorrichtung angeordnet, die dazu ausgebildet ist, den Hauptkörper auf eine vorbestimmte Betriebstemperatur zu heizen und auf dieser zu halten, beispielsweise bei ca. 850°C.

Die JP 2019/203837 A betrifft einen Stickoxidsensor mit zusätzliche Mischpotentialelektrode zur Ammoniakmessung. Der daraus bekannte Stickoxidsensor umfasst zudem einen Eigendiagnosebetrieb, um die Ammoniakmessung zu überwachen. Hierzu wird der Leistungswert durch Differenzbildung zwischen dem mittels Ammoniaksensor ermittelten Ammoniakwert und dem mittels Stickoxidsensor abgeschätzten Ammoniakwert ermittelt.

Die US 2020/0088665 A1 offenbart eine Diagnosevorrichtung für Gassensoren. Hier wird während einer Schubphase eine Untersuchung des Ammoniak-Drifts gemacht. Dabei wird sich zu Nutze gemacht, dass die Heiztemperatur des Sensorelements umso höher ist, je größer die Elektrodenalterung ist.

Aus der US 10 527 569 B2 ist ein Verfahren zum Ermitteln der Alterung einer Mischpotentialelektrode bekannt.

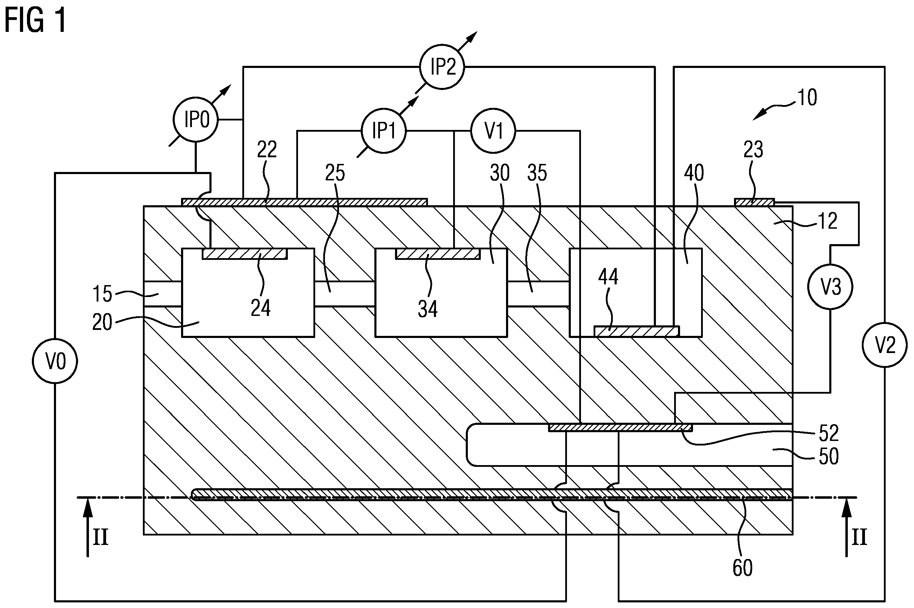

Die DE 199 82 982 B4 offenbart einen Gassensor mit Selbstdiagnose bzw. ein Verfahren zur Selbstdiagnose. Nach Abbildung 1 umfasst der Gassensor einen Hauptkörper mit einer Sauerstoffpumpzelle zur Steuerung einer Sauerstoffkonzentration in einer ersten Gaskammer. Zur Messung der dort vorhandenen Sauerstoffkonzentration existiert eine Sauerstoffmesselektrode. In einer zweiten Gaskammer existiert eine zweite Sauerstoffmesselektrode für die zu erfassende Gasatmosphäre. Es wird jeweils ein Sauerstoffkonzentrationssignal in der ersten Gaskammer und in der zu erfassenden Gasatmosphäre ausgelesen (zweite Gaskammer). Diese beiden Signale werden verglichen und zur Diagnose herangezogen. Die Druckschrift offenbart keine Mischpotentialelektrode an der Außenseite des Hauptkörpers mit der ein Ammoniakwert ermittelt wird und somit ein Ammoniakschlupf erfasst wird.

Die DE 10 2019 004 190 A1 beschreibt einen Gassensor und ein Gaskonzentrationsmessverfahren.

Der vorliegenden Erfindung liegt die Aufgabe zu Grunde, ein Verfahren und einen Abgassensor bereitzustellen, mit dem ein Fehler des Abgassensors ermittelt werden kann.

Diese Aufgabe wird mit einem Verfahren gemäß unabhängigem Anspruch 1 und einem Abgassensor gemäß unabhängigem 9 gelöst. Vorteilhafte Ausgestaltungen sind in den Unteransprüchen angegeben.

Der vorliegenden Erfindung im Wesentlichen der Gedanke zugrunde, ein Verfahren zur Eigendiagnose eines Abgassensors, insbesondere eines Stickoxidsensors mit erweiterter Ammoniakmessfunktion, bereitzustellen. Insbesondere macht sich die Erfindung dabei zu Nutze, dass ein Stickoxidsensor mit zusätzlicher Mischpotentialelektrode zur Ammoniakmessung die Fähigkeit aufweist, zwei unabhängig voneinander erfasste Sauerstoffwerte zu ermitteln, die dann zur Eigendiagnose miteinander verglichen werden können. Bei einer Abweichung der beiden ermittelten Sauerstoffwerte voneinander um mehr als einen vorbestimmten Sauerstoffschwellenwert kann dann einen Fehler des Abgassensors festgestellt werden. Genauer gesagt kann dabei der während eines vorbestimmten Betriebszustands der Brennkraftmaschine vom Stickoxidsensor ermittelte Sauerstoffwert mit dem mittels der Mischpotentialelektrode ermittelten Sauerstoffwert miteinander vergleichen und bei Abweichen der beiden ermittelten Sauerstoffwerte voneinander einen Fehler des Abgassensors, insbesondere der Mischpotentialelektrode, festgestellt werden.

Folglich ist gemäß einem ersten Aspekt der vorliegenden Erfindung ein Verfahren zum Ermitteln eines Fehlers eines einen Hauptkörper aufweisenden Abgassensors offenbart, der dazu ausgebildet ist, in einem Abgasstrang einer Brennkraftmaschine angeordnet zu werden. Der Abgassensor weist eine im Hauptkörper angeordnete und mit dem Abgas verbundene Pumpkavität, in der eine Pumpelektrode angeordnet ist, eine an der Außenseite des Hauptkörpers angeordnete und mit dem Abgas in Kontakt tretende Mischpotentialelektrode und eine im Hauptkörper angeordnete und mit der Umgebungsluft verbundene Referenzkavität auf, in der eine Referenzelektrode angeordnet ist. Das erfindungsgemäße Verfahren umfasst ein Ermitteln eines vorbestimmten Betriebszustandes der Brennkraftmaschine, in dem das Abgas im Wesentlichen ammoniakfrei ist, ein Ermitteln eines ersten Sauerstoffwerts basierend auf einem derart an der Pumpelektrode angelegten Pumpstrom, dass eine sich zwischen der Pumpelektrode und der Referenzelektrode ausbildende Elektrodenspannung auf einem vorbestimmten Spannungswert konstant gehalten wird, ein Ermitteln eines zweiten Sauerstoffwerts basierend auf einer sich zwischen der Mischpotentialelektrode und der Referenzelektrode ausbildenden Mischpotentialspannung und ein Ermitteln eines Fehlers des Abgassensors, wenn der erste Sauerstoffwert vom zweiten Sauerstoffwert um mehr als einen vorbestimmten Sauerstoffschwellenwert abweicht. Das erfindungsgemäße Verfahren umfasst zudem ein Ermitteln eines Ammoniakwerts basierend auf einer sich zwischen der Mischpotentialelektrode und der Referenzelektrode ausbildenden Mischpotentialspannung und ein Ermitteln eines Ammoniakschlupfs im Abgasstrang der Brennkraftmaschine, wenn der ermittelte Ammoniakwert größer ist als ein vorbestimmter Ammoniakschwellenwert.

Somit kann durch einen Vergleich des mittels dem an der Pumpelektrode angelegten Pumpstrom ermittelten ersten Sauerstoffwerts mit dem sich auf der Grundlage der Mischpotentialspannung ermittelten, zweiten Sauerstoffwert eine Diagnose des Abgassensors, insbesondere der Mischpotentialelektrode, durchgeführt werden. Weicht dabei der zweite Sauerstoffwert um mehr als den vorbestimmten Sauerstoffschwellenwert vom ersten Sauerstoffwert ab, kann ein Fehler der Mischpotentialelektrode festgestellt werden. Vor allem kann vorausgesetzt werden, dass aufgrund der routinemäßigen Eigendiagnose des Stickoxidsensors und somit der Pumpelektrode der erste Sauerstoffwert fehlerfrei sein sollte und folglich als Vergleichsmaß für den zweiten Sauerstoffwert herangezogen werden kann.

Bevorzugt wird ein Fehler der Mischpotentialelektrode ermittelt, wenn der erste Sauerstoffwert vom zweiten Sauerstoffwert um mehr als den vorbestimmten Sauerstoffschwellenwert abweicht. Ein Fehler der Mischpotentialelektrode liegt beispielsweise dann vor, wenn die Mischpotentialelektrode übermäßig gealtert ist. Zudem kann ein Fehler der Mischpotentialelektrode vorliegen, wenn die Mischpotentialelektrode vergiftet ist und somit weniger oder nicht mehr sensitiv ist.

Vorzugsweise beträgt der vorbestimmte Ammoniakschwellenwert 1 ppm.

Wenn der Abgassensor also einen Ammoniakwert anzeigt, der größer ist als der vorbestimmte Ammoniakschwellenwert, kann davon ausgegangen werden, dass es zu einem erhöhten Ammoniakschlupf durch den SCR-Katalysator kommt, der vom Abgassensor detektiert werden kann.

In einer weiteren bevorzugten Ausgestaltung umfasst das erfindungsgemäße Verfahren zusätzlich ein Feststellen, dass ein Ermitteln eines Fehlers des Abgassensors nicht möglich ist, wenn der ermittelte erste Sauerstoffwert von einem Sauerstoffreferenzwert um mehr als einen vorbestimmten Sauerstoffschwellenwert abweicht. Der Sauerstoffreferenzwert liegt dabei bevorzugt in einem Bereich zwischen ungefähr 20 % und ungefähr 21%, was dem ungefähren regulären Anteil an Sauerstoff in der Luft entspricht.

Wenn der erste Sauerstoffwert von dem Sauerstoffreferenzwert um mehr als einen Sauerstoffschwellenwert, wie beispielsweise 1%, abweicht, ist somit ein Eigendiagnoseverfahren gemäß der vorliegenden Erfindung nicht mehr ordnungsgemäß durchführbar, da wohl das Abgas nicht ammoniakfrei ist und somit eine Diagnose aufgrund der Querempfindlichkeit der Mischpotentialelektrode nicht möglich ist.

Bei dem vorbestimmten Betriebszustand der Brennkraftmaschine handelt es sich in vorteilhafter Weise um einen Schub oder einen Motornachlauf. Ferner kann ein überhitzter SCR-Katalysator einen ammoniakfreien SCR-Katalysator und somit einen vorbestimmten Betriebszustand der Brennkraftmaschine anzeigen, beispielsweise wenn über einen längeren Zeitraum hinweg kein Reduktionsmittel eingespritzt wurde. Insbesondere ist während dieser Betriebszustände der Brennkraftmaschine das Abgas im Wesentlichen ammoniakfrei, weshalb die Mischpotentialelektrode, die auf Sauerstoff querempfindlich ist, lediglich auf den Sauerstoff im Abgas empfindlich ist. Somit kann das Signal der Mischpotentialelektrode dazu dienen, den Sauerstoffgehalt im Abgas bestimmen zu können und mit dem mittels der Pumpelektrode ermittelten ersten Sauerstoffwert zu vergleichen.

In vorteilhafter Weise beträgt der vorbestimmte Sauerstoffschwellenwert ungefähr 2%, vorzugsweise ungefähr 1,5% und noch bevorzugter ungefähr 1%. In weiteren bevorzugten Ausgestaltungen kann der vorbestimmte Sauerstoffschwellenwert ungefähr 0,5 % betragen.

Gemäß einem weiteren Aspekt der vorliegenden Erfindung ist ein Abgassensor zum Anordnen in einem Abgasstrang einer Brennkraftmaschine offenbart. Der erfindungsgemäße Abgassensor weist einen Hauptkörper, eine im Hauptkörper angeordnete und mit dem Abgas verbundene Pumpkavität, in der eine Pumpelektrode angeordnet ist, eine an der Außenseite des Hauptkörpers angeordnete und mit dem Abgas in Kontakt tretende Mischpotentialelektrode, eine im Hauptkörper angeordnete und mit der Umgebungsluft verbundene Referenzkavität, in der eine Referenzelektrode angeordnet ist, und eine Steuerungseinheit auf, die dazu ausgebildet ist, ein erfindungsgemäßes Verfahren zum Ermitteln eines Fehlers des Abgassensors durchzuführen.

Weitere Merkmale und Aufgaben der Erfindung werden dem Fachmann durch Ausüben der vorliegenden Lehre und Betrachten der beiliegenden Zeichnungen ersichtlich, in denen:
- Fig. 1: eine schematische Schnittansicht durch einen Abgassensor für eine Brennkraftmaschine eines Fahrzeugs zeigt,
und
- Fig. 2: ein beispielhaftes Ablaufdiagramm eines Verfahrens zum Ermitteln eines Fehlers eines Abgassensors für eine Brennkraftmaschine zeigt.

Im Rahmen der vorliegenden Offenbarung sind amperometrisch arbeitende Sensoren, wie beispielsweise Stickoxidsensoren, Lambdasonden und Sauerstoffsensoren, dadurch gekennzeichnet, dass deren Messprinzip auf der Amperometrie basiert, d. h. auf einer elektrochemischen Methode zur quantitativen Bestimmung von chemischen Stoffen. Insbesondere wird an einer Arbeitselektrode ein elektrischer Strom derart eingestellt, dass sich ein zeitlich konstantes und vorbestimmtes elektrochemisches Potential einstellt.

Im Rahmen der vorliegenden Offenbarung betreffen Mischpotentialsensoren Sensoren, bei denen sich zwischen zwei Elektroden aufgrund elektrochemischer Prozesse ein elektrisches Potential ausbildet, welches als Maß für eine Zusammensetzung desjenigen Gases herangezogen werden kann, dem die Elektroden ausgesetzt sind. Hierbei kommt es, wie bei amperometrischen Sensoren, zur sogenannten Nernstspannung.

Ferner umfasst im Rahmen der vorliegenden Offenbarung der Begriff "Steuerung" die regelungstechnischen Begriffe "Steuern" und "Regeln". Der Fachmann wird jeweils erkennen, wann ein regelungstechnisches Steuern und wann ein regelungstechnisches Regeln anzuwenden ist.

Die Fig. 1 zeigt einen beispielhaften Abgassensor 10, wie beispielsweise einen Stickoxidsensor mit zusätzlicher Mischpotentialelektrode 23 zur Ammoniakmessung. Ferner ist die vorliegende Erfindung bei Abgassensoren 10 anwendbar, die einen keramischen Grundträger mit daran angebrachten Elektroden zur Sauerstoffmessung aufweisen.

Unter Verweis auf die Fig. 1 ist eine schematische Schnittansicht eines beispielhaften Stickoxidsensor 10 dargestellt, der dazu ausgebildet ist, in einem Abgasstrang einer Brennkraftmaschine (nicht gezeigt) angeordnet zu werden und den Stickoxidgehalt bzw. den Sauerstoffgehalt im Abgas der Brennkraftmaschine quantitativ zu erfassen.

Der Stickoxidsensor 10 weist einen Hauptkörper 12 aus einem Feststoffelektrolyten auf, der vorzugsweise aus einem Mischkristall aus Zirkonoxid und Yttriumoxid und/oder durch einen Mischkristall aus Zirkonoxid und Calciumoxid gebildet ist. Zusätzlich kann ein Mischkristall aus Hafniumoxid, ein Mischkristall aus Perowskit-basierten Oxiden oder ein Mischkristall aus trivalentem Metalloxid verwendet werden, wie beispielsweise Aluminiumoxid (Al₂O₃). Der Hauptkörper 12 bildet ein Sensorelement des Abgassensors 10.

Innerhalb des Hauptkörpers 12 des exemplarisch dargestellten Stickoxidsensors sind eine erste Pumpkavität 20, eine zweite Pumpkavität 30 und eine Messkavität 40 vorgesehen. Die erste Pumpkavität 20 ist über einen Verbindungspfad 15 mit dem Äußeren des Hauptkörpers 12 verbunden. Insbesondere kann Abgas durch den Verbindungspfad 15 in die erste Pumpkavität 20 strömen Die zweite Pumpkavität 30 ist mit der ersten Pumpkavität 20 über einen ersten Diffusionspfad 25 verbunden.

Die Messkavität 40 ist mit der zweiten Pumpkavität 30 über einen zweiten Diffusionspfad 35 verbunden.

Im Hauptkörper 12 ist außerdem eine Referenzkavität 50 gebildet, die direkt mit dem Äußeren des Hauptkörpers 12 in Verbindung steht. Innerhalb der Referenzkavität 50 ist eine Referenzelektrode 52 angeordnet. Insbesondere steht die Referenzkavität 50 mit der Umgebungsluft, d. h. nicht mit dem Abgas, in Verbindung und ist dazu ausgebildet, eine Sauerstoffreferenz für die im Stickoxidsensor angeordneten verschiedenen Elektroden zu bilden.

An einer Außenseite des Hauptkörpers 12 ist eine Abgaselektrode 22 angeordnet. Insbesondere kann während eines Messbetriebs des Stickoxidsensors durch Anlegen eines Referenzstroms an der Abgaselektrode 22 der im Abgas befindliche Sauerstoff ionisiert werden und durch den Hauptkörper 12 als Sauerstoffionen zur Referenzelektrode 52 diffundieren und dort wieder in Sauerstoffmoleküle zur Ausbildung einer Sauerstoffreferenz umgewandelt werden.

Innerhalb der ersten Pumpkavität 20 ist eine erste Pumpelektrode 24 angeordnet. Insbesondere kann während des Messbetriebs des Stickoxidsensors durch Anlegen eines ersten Pumpstroms IP0 an der ersten Pumpelektrode 24 der im Abgas befindliche Sauerstoff innerhalb der ersten Pumpkavität 20 ionisiert werden und durch den Hauptkörper 12 als Sauerstoffionen wandern bzw. gelangen. Aufgrund der aus der ersten Pumpkavität 20 ausgebrachten Sauerstoffionen bildet sich zwischen der ersten Pumpelektrode 24 und der Referenzelektrode 52 indirekt eine erste Elektrodenspannung bzw. erste Nernstspannung V0 aus. Genauer gesagt bildet sich die erste Elektrodenspannung bzw. die erste Nernstspannung V0 direkt aus dem in der ersten Pumpkavität 20 noch vorliegenden Rest-Sauerstoff.

Innerhalb der zweiten Pumpkavität 30 ist eine zweite Pumpelektrode 34 angeordnet. Hier kann während des Messbetriebs des Stickoxidsensors durch Anlegen eines zweiten Pumpstroms IP1 an der zweiten Pumpelektrode 34 der im Gasgemisch befindliche Sauerstoff innerhalb der zweiten Pumpkavität 30 ionisiert werden und durch den Hauptkörper 12 als Sauerstoffionen wandern bzw. gelangen. Aufgrund der aus der zweiten Pumpkavität 30 ausgebrachten Sauerstoffionen bildet sich zwischen der zweiten Pumpelektrode 34 und der Referenzelektrode 52 indirekt eine zweite Elektrodenspannung bzw. zweite Nernstspannung V1 aus. Genauer gesagt bildet sich die zweite Elektrodenspannung bzw. die zweite Nernstspannung V1 direkt aus dem in der zweiten Pumpkavität 30 noch vorliegenden Rest-Sauerstoff.

Innerhalb der Messkavität 40 ist eine Messelektrode 44 angeordnet, die dazu ausgebildet ist, während des Messbetriebs des Stickoxidsensors bei Anlegen eines Messstroms IP2 den innerhalb der Messkavität 40 vorhandenen Sauerstoff und/oder Stickoxide zu ionisieren, so dass die Sauerstoffionen durch den Hauptkörper 12 wandern bzw. gelangen können. Aufgrund der aus der Messkavität 40 ausgebrachten bzw. herausgepumpten Sauerstoffionen bildet sich zwischen der Messelektrode 44 und der Referenzelektrode 52 eine dritte Elektrodenspannung bzw. dritte Nernstspannung V2 aus, die durch Anlegen des Messstroms IP2 an der Messelektrode 44 auf konstantem Wert gehalten wird. Genauer gesagt bildet sich die dritte Elektrodenspannung bzw. die dritte Nernstspannung V2 direkt aus dem in der Messkavität 40 noch vorliegenden Rest-Sauerstoff. Der angelegte Messstrom IP2 ist dann ein Indiz für den innerhalb des Abgases befindlichen Stickoxidgehalt.

Die an der ersten und zweiten Pumpelektrode 24, 34 anliegenden Pumpströme IP0, IP1 sind derart eingestellt, das bevorzugt nur der Sauerstoff ionisiert wird, jedoch nicht die Stickoxide. Insbesondere ist die erste Pumpelektrode 24 dazu ausgebildet, während des Normalbetriebs des Stickoxidsensors nahezu den gesamten Sauerstoff aus dem Abgas zu pumpen bzw. einen vorbestimmten Sauerstoffschlupf aus der ersten Pumpkavität 20 in die zweite Pumpkavität 30 zuzulassen. Die zweite Pumpelektrode 34 ist dazu ausgebildet, den aus der ersten Pumpkavität 20 noch nicht herausgepumpten Sauerstoff zu ionisieren und abzuleiten, so dass in der Messkavität 40 nahezu nur Stickoxide vorliegen. Die Messelektrode 44 ist dazu ausgebildet, die Stickoxide zu ionisieren, wobei der an der Messelektrode 44 angelegte Messstrom IP2 ein Maß für den Stickoxidgehalt im Abgas ist.

Innerhalb des Hauptkörpers 12 ist ferner eine Heizvorrichtung 60 angeordnet, die dazu ausgebildet ist, den Hauptkörper 12 auf eine vorbestimmte Betriebstemperatur zu heizen und auf dieser zu halten, beispielsweise bei ca. 850°C.

Die Betriebsweise zum Ermitteln des Stickoxidgehalts im Abgas der Brennkraftmaschine mittels des offenbarten Stickoxidsensors ist bereits aus dem Stand der Technik bekannt, auf den an dieser Stelle verwiesen wird. Das regelungstechnische Steuerungsprinzip für den Stickoxidsensor der Fig. 1 ist nämlich dadurch gekennzeichnet, dass die jeweiligen Elektrodenspannungen bzw. Nernstspannungen V0, V1, V2 durch Anlegen und Anpassen der Pumpströme IP0, IP1 und des Messstroms IP2 auf konstantem Niveau gehalten werden.

Der Abgassensor 10 der Fig. 1 weist ferner die Funktionalität der Ermittlung des Ammoniakgehalts (NH3-Gehalts) im Abgas der Brennkraftmaschine auf. Hierzu umfasst der Abgassensor 10 der Fig. 1 zusätzlich eine Mischpotentialelektrode (oder Messelektrode) 23, die an einer Außenseite des Hauptkörpers 12 angeordnet ist und mit dem Abgas der Brennkraftmaschine in direkten Kontakt treten kann. Die Mischpotentialelektrode 23 ist insbesondere auf Ammoniak empfindlich, weist aber auch eine Querempfindlichkeit auf andere Fettgase auf, wie beispielsweise Kohlenwasserstoffe, Kohlenmonoxid und Wasserstoff. Das heißt, dass mit dem im Abgassensor 10 integrierten Mischpotentialsensor, der die Mischpotentialelektrode 23 umfasst, aus dem Ausgangssignal (= Nernstspannung V3, siehe unten) unter Anwendung eine Funktion der Ammoniakgehalt und Sauerstoffgehalt im Abgas erfasst werden kann.

Während des Betriebs des Abgassensors 10 bildet sich aufgrund der elektrochemischen Prozesse im Hauptkörper 12 eine dritte Nernstspannung bzw. Elektrodenspannung (im Folgenden auch Mischpotentialspannung genannt) V3 zwischen der Mischpotentialelektrode 23 und der Referenzelektrode 52 aus, die zum Ermitteln der Sauerstoffkonzentration im Abgas und der Ammoniakkonzentration im Abgas ausgebildet werden kann. Zur Auswertung der Mischpotentialspannung V3 können Analyseverfahren herangezogen werden, mit Hilfe deren der Sauerstoffwert und der Ammoniakwert getrennt und separat ermittelt werden können, wobei ein erstes Analyseverfahren zur Ausgabe des Ammoniakwerts im Abgas führt und ein zweites Analyseverfahren zur Ausgabe des Sauerstoffwerts im Abgas führt. Insbesondere handelt es sich bei der Ermittlung des zweiten Sauerstoffwerts mittels der Mischpotentialspannung V3 um eine vom ersten Sauerstoffwert ermittelten unabhängigen Sauerstoffwert und kann separat erfolgen. Insbesondere wird hierzu werkseitig das entsprechende Verfahren softwaremäßig implementiert und kann dann während dem Betrieb dazu beitragen, die Genauigkeit des Abgassensors 10 zu erhöhen.

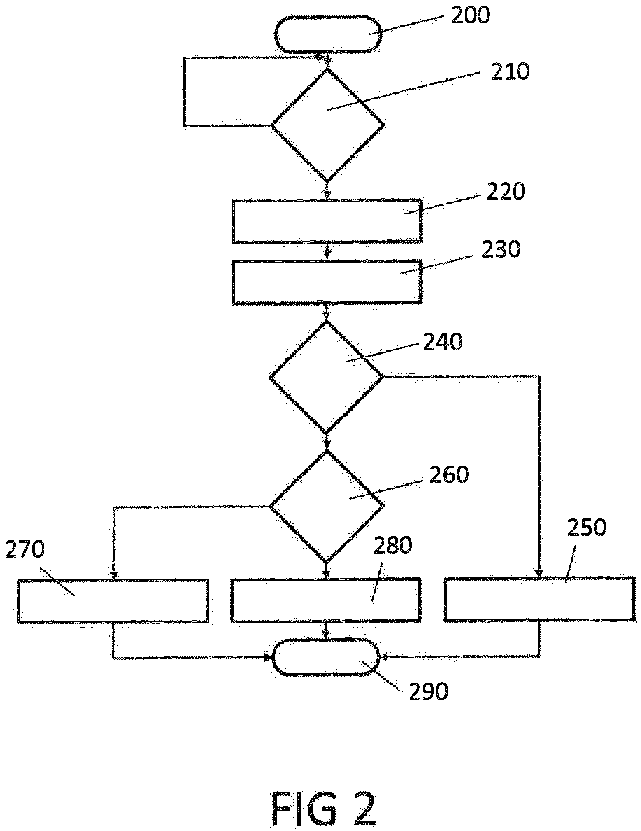

Die Fig. 2 zeigt ein beispielhaftes Ablaufdiagramm eines erfindungsgemäßen Verfahrens zum Ermitteln eines Fehlers eines Abgassensors, wie beispielsweise des Abgassensor 10 der Fig. 1.

Das Verfahren der Fig. 2 startet beim Schritt 200 und gelangt dann zum Schritt 210, an dem bestimmt wird, ob sich die Brennkraftmaschine in einem vorbestimmten Betriebszustand befindet. Das Ermitteln, ob sich die Brennkraftmaschine in einem vorbestimmten Betriebszustand befindet, erfolgt bevorzugt in der Steuerung der Brennkraftmaschine, wobei die Steuerung ein dem ermittelten Betriebszustand anzeigendes Signal dem Abgassensor 10 bereitstellt. Der vorbestimmte Betriebszustand ist dadurch gekennzeichnet, dass das Abgas während diesem Betriebszustand im Wesentlichen ammoniakfrei ist. Beispielsweise kann der Schubzustand oder der Motornachlauf als vorbestimmter Betriebszustand der Brennkraftmaschine betrachtet werden, da während diesen Betriebszuständen das Abgas im Wesentlichen ammoniakfrei ist. Das Verfahren verbleibt solange beim Schritt 210, bis ein vorbestimmter Betriebszustand ermittelt worden ist.

Wird beim Schritt 210 ermittelt, dass sich die Brennkraftmaschine in einem vorbestimmten Betriebszustand befindet, gelangt das Verfahren zum Schritt 220, an dem ein erster Sauerstoffwert basierend auf dem an der Pumpelektrode 42 angelegten Pumpstrom IP0 ermittelt wird.

In einem darauffolgenden Schritt 230 wird ein zweiter Sauerstoffwert basierend auf der sich zwischen der Mischpotentialelektrode und der Referenzelektrode ausbildenden Mischpotentialspannung V3 ermittelt. Bevorzugt laufen die Schritte 220 und 230 zeitgleich ab.

In einem darauffolgenden Schritt 240 wird geprüft, ob der ermittelte erste Sauerstoffwert die Eignung aufweist, als Vergleichswert für den ermittelten zweiten Sauerstoffwert zu dienen. Insbesondere wird beim Schritt 240 überprüft, ob der ermittelte erste Sauerstoffwert von einem Sauerstoffreferenzwert um nicht mehr als einen vorbestimmten Sauerstoffschwellenwert abweicht. Insbesondere kann beim Schritt 240 also überprüft werden, ob der erste Sauerstoffwert den erwarteten ungefähr 21% Sauerstoffanteil in der Luft entspricht. Falls beim Schritt 240 festgestellt wird, dass der ermittelte erste Sauerstoffwert von dem Sauerstoffreferenzwert um mehr als einen Sauerstoffschwellenwert abweicht, gelangt das Verfahren zum Schritt 250, an dem festgestellt wird, dass ein Ermitteln eines Fehlers des Abgassensors 10 gemäß der vorliegenden Erfindung nicht möglich ist, bevor das Verfahren beim Schritt 290 endet. Ist das Abgas nämlich nicht ammoniakfrei, kann nicht mit ausreichend hoher Sicherheit ausgesagt werden, ob die Mischpotentialelektrode 23 auf den im Abgas befindlichen Sauerstoff oder auf das im Abgas befindliche Ammoniak reagiert hat.

Wird jedoch beim Schritt 240 bestimmt, dass der ermittelte erste Sauerstoffwert von dem Sauerstoffreferenzwert um nicht mehr als einen vorbestimmten Schwellenwert abweicht, gelangt das Verfahren zum Schritt 260, an dem der ermittelte erste Sauerstoffwert mit dem ermittelten zweiten Sauerstoffwert verglichen wird. Insbesondere wird beim Schritt 260 überprüft, ob der erste Sauerstoffwert vom zweiten Sauerstoffwert um mehr als einen vorbestimmten Sauerstoffschwellenwert abweicht.

Wird beim Schritt 260 ermittelt, dass der erste Sauerstoffwert vom zweiten Sauerstoffwert um nicht mehr als einen vorbestimmten Sauerstoffschwellenwert abweicht, gelangt das Verfahren zum Schritt 270, an dem ein fehlerfreier Abgassensor 10, insbesondere eine fehlerfreie Mischpotentialelektrode 23, ermittelt wird, bevor das Verfahren beim Schritt 290 wieder endet.

Wird jedoch beim Schritt 260 ermittelt, dass der erste Sauerstoffwert vom zweiten Sauerstoffwert um mehr als den vorbestimmten Sauerstoffschwellenwert abweicht, gelangt das Verfahren zum Schritt 280, an dem ein Fehler des Abgassensors 10, insbesondere der Mischpotentialelektrode 23, ermittelt wird, bevor das Verfahren wiederum beim Schritt 290 endet.

Gemäß der vorliegenden Erfindung kann also ein Abgassensor hinsichtlich seiner Funktionalität überprüft werden. Insbesondere kann dabei ein Stickoxidsensor, der mittels einer zusätzlichen Mischpotentialelektrode 23 um die Funktionalität einer Ammoniakmessung erweitert ist, dahingehend überprüft werden, ob die Mischpotentialelektrode 23 des Stickoxidsensors 10 einwandfrei funktioniert. Bevorzugt werden den elektrischen Signalen des Abgassensors 10 zunächst die jeweiligen Konzentrationswerte zugeordnet, die dann miteinander verglichen werden könnten. Das heißt, dass beispielsweise auf der Basis der Mischpotentialspannung V3 zunächst gemäß ihrer standardmäßigen Zuordnungsvorschrift der Sauerstoffwert und/oder der Ammoniakwert ermittelt wird, bevor ein jeweiliger Vergleich mit Referenzwerten oder anderen Werten erfolgt. Gleiches gilt für den Pumpstrom IP0, der zunächst auf den entsprechenden ersten Sauerstoffwert umgerechnet wird, bevor ein Vergleich mit anderen ermittelten Sauerstoffwerten erfolgen kann.

Die in der Fig. 2 dargestellte Reihenfolge der Verfahrensschritte ist nicht auf das gezeigte Beispiel beschränkt, insbesondere kann sich der Schritt 240 direkt an den Schritt 220 anschließen, so dass der zweite Sauerstoffwert erst dann ermittelt wird, wenn eine Eignung des ersten Sauerstoffwerts zur Fehlerdiagnose des Mischpotentialsensors vorliegt.

## Patentansprüche

1. Verfahren zum Ermitteln eines Fehlers eines einen Hauptkörper (12) aufweisenden Abgassensors (10), der dazu ausgebildet ist, in einem Abgasstrang einer Brennkraftmaschine angeordnet zu werden, wobei der Abgassensor (10) eine im Hauptkörper (12) angeordnete und mit dem Abgas verbundene Pumpkavität (20), in der eine Pumpelektrode (24) angeordnet ist, eine an der Außenseite des Hauptkörpers (12) angeordnete und mit dem Abgas in Kontakt tretende Mischpotentialelektrode (23), und eine im Hauptkörper (12) angeordnete und mit der Umgebungsluft verbundene Referenzkavität (50) aufweist, in der eine Referenzelektrode (52) angeordnet ist, wobei das Verfahren aufweist:
- Ermitteln eines vorbestimmten Betriebszustands der Brennkraftmaschine, in dem das Abgas im Wesentlichen ammoniakfrei ist,
- Ermitteln eines ersten Sauerstoffwerts basierend auf einem derart an der Pumpelektrode (24) angelegten Pumpstrom (IP0), dass eine sich zwischen der Pumpelektrode (24) und der Referenzelektrode (52) ausbildende Elektrodenpumpspannung (V0) auf einem vorbestimmten Spannungswert konstant gehalten wird,
- Ermitteln eines zweiten Sauerstoffwerts basierend auf einer sich zwischen der Mischpotentialelektrode (23) und der Referenzelektrode (52) ausbildenden Mischpotentialspannung (V3),
- Ermitteln eines Fehlers des Abgassensors (10), wenn der erste Sauerstoffwert vom zweiten Sauerstoffwert um mehr als einen vorbestimmten Sauerstoffschwellenwert abweicht,
- Ermitteln eines Ammoniakwerts basierend auf einer sich zwischen der Mischpotentialelektrode (23) und der Referenzelektrode (52) ausbildenden Mischpotentialspannung (V3), und
- Ermitteln eines Ammoniakschlupfs im Abgasstrang der Brennkraftmaschine, wenn der ermittelte Ammoniakwert größer ist als ein vorbestimmter Ammoniakschwellenwert.

2. Verfahren nach Anspruch 1, wobei ein Fehler der Mischpotentialelektrode (23) ermittelt wird, wenn der erste Sauerstoffwert vom zweiten Sauerstoffwert um mehr als den vorbestimmten Sauerstoffschwellenwert abweicht.

3. Verfahren nach einem der vorhergehenden Ansprüche, wobei der vorbestimmte Ammoniakschwellenwert ungefähr 1 ppm beträgt.

4. Verfahren nach einem der vorhergehenden Ansprüche, ferner mit:
- Feststellen, dass ein Ermitteln eines Fehlers des Abgassensor (10) nicht möglich ist, wenn der ermittelte erste Sauerstoffwert von einem Sauerstoffreferenzwert um mehr als einen vorbestimmten Sauerstoffschwellenwert abweicht.

5. Verfahren nach Anspruch 4, wobei der Sauerstoffreferenzwert in einem Bereich zwischen ungefähr 20 % und ungefähr 21 % liegt.

6. Verfahren nach einem der vorhergehenden Ansprüche, wobei der vorbestimmte Betriebszustands der Brennkraftmaschine einen Schub oder Nachlauf aufweist.

7. Verfahren nach einem der vorhergehenden Ansprüche, wobei der vorbestimmte Sauerstoffschwellenwert ungefähr 2%, vorzugsweise ungefähr 1,5 % und am bevorzugtesten ungefähr 1 % beträgt.

8. Abgassensor (10) zum Anordnen in einem Abgasstrang einer Brennkraftmaschine, wobei der Abgassensor (10) aufweist:
- einen Hauptkörper,
- eine im Hauptkörper (12) angeordnete und mit dem Abgas verbundene Pumpkavität (20), in der eine Pumpelektrode (24) angeordnet ist,
- eine an der Außenseite des Hauptkörpers (12) angeordnete und mit dem Abgas in Kontakt tretende Mischpotentialelektrode (23),
- eine im Hauptkörper (12) angeordnete und mit der Umgebungsluft verbundene Referenzkavität (50), in der eine Referenzelektrode (52) angeordnet ist, und
- eine Steuerungseinheit, die dazu ausgebildet ist, ein Verfahren nach einem der vorhergehenden Ansprüche auszuführen.

## Claims

1. Method for ascertaining a fault of an exhaust gas sensor (10), which comprises a main body (12) and is configured to be arranged in an exhaust gas tract of an internal combustion engine, wherein the exhaust gas sensor (10) comprises a pump cavity (20)being arranged in the main body (12) and being connected to the exhaust gas, a pump electrode (24) being arranged in said pump cavity, a mixed potential electrode (23) being arranged on the outer side of the main body (12) and coming into contact with the exhaust gas, and a reference cavity (50) being arranged in the main body (12) and being connected to ambient air, wherein a reference electrode (52) being arranged in said reference cavity (50), the method comprising:
- ascertaining a predetermined internal combustion engine operating state in which the exhaust gas is substantially free of ammonia,
- ascertaining a first oxygen value on the basis of a pump current (IP0) applied to the pump electrode (24) in such a way that an electrode pump voltage (V0) formed between the pump electrode (24) and the reference electrode (52) is kept constant at a predetermined voltage value,
- ascertaining a second oxygen value on the basis of a mixed potential voltage (V3) formed between the mixed potential electrode (23) and the reference electrode (52),
- ascertaining a fault of the exhaust gas sensor (10) if the first oxygen value differs from the second oxygen value by more than a predetermined oxygen threshold value,
- ascertaining an ammonia value on the basis of a mixed potential voltage (V3) formed between the mixed potential electrode (23) and the reference electrode (52), and
- ascertaining ammonia slip in the exhaust gas tract of the internal combustion engine if the ammonia value ascertained is greater than a predetermined ammonia threshold value.

2. Method according to Claim 1, wherein a fault of the mixed potential electrode (23) is ascertained if the first oxygen value differs from the second oxygen value by more than the predetermined oxygen threshold value.

3. Method according to any one of the preceding claims, wherein the predetermined ammonia threshold value is approximately 1 ppm.

4. Method according to any one of the preceding claims, further comprising:
- determining that it is not possible to ascertain a fault of the exhaust gas sensor (10) if the first oxygen value ascertained differs from an oxygen reference value by more than a predetermined oxygen threshold value.

5. Method according to Claim 4, wherein the oxygen reference value is in a range between approximately 20 % and approximately 21 %.

6. Method according to any one of the preceding claims, wherein the predetermined internal combustion engine operating state includes fuel cut-off or run-on.

7. Method according to any one of the preceding claims, wherein the predetermined oxygen threshold value is approximately 2 %, preferably approximately 1.5 % and most preferably approximately 1 %.

8. Exhaust gas sensor (10) for arrangement in an exhaust gas tract of an internal combustion engine, the exhaust gas sensor (10) comprising:
- a main body,
- a pump cavity (20) being arranged in the main body (12) and being connected to the exhaust gas, a pump electrode (24) being arranged in said pump cavity,
- a mixed potential electrode (23) being arranged on an outer side of the main body (12) and coming into contact with the exhaust gas,
- a reference cavity (50) being arranged in the main body (12) and being connected to ambient air, a reference electrode (52) being arranged in said reference cavity, and
- a control unit being configured to execute a method according to any one of the preceding claims.

## Revendications

1. Procédé de détermination d'une défaillance d'un capteur (10) de gaz d'échappement comprenant un corps principal (12) et conçu pour être agencé dans une ligne d'échappement d'un moteur à combustion interne, le capteur (10) de gaz d'échappement comprenant une cavité de pompage (20) ménagée dans le corps principal (12) et reliée aux gaz d'échappement, dans laquelle est agencée une électrode de pompage (24), une électrode (23) à potentiel mixte agencée à l'extérieur du corps principal (12) et étant en contact avec les gaz d'échappement, et une cavité de référence (50) ménagée dans le corps principal (12) et reliée à l'air ambiant, dans laquelle est agencée une électrode de référence (52), le procédé comprenant :
- le fait de déterminer un état de fonctionnement prédéterminé du moteur à combustion interne dans lequel les gaz d'échappement sont essentiellement exempts d'ammoniac,
- le fait de déterminer une première valeur d'oxygène sur la base d'un courant de pompage (IP0) appliqué à l'électrode de pompage (24) de telle sorte qu'une tension (V0) de pompage d'électrode formée entre l'électrode de pompage (24) et l'électrode de référence (52) soit maintenue constante à une valeur de tension prédéterminée,
- le fait de déterminer une deuxième valeur d'oxygène sur la base d'une tension (V3) de potentiel mixte formée entre l'électrode (23) de potentiel mixte et l'électrode de référence (52),
- le fait de déterminer une défaillance du capteur (10) de gaz d'échappement lorsque la première valeur d'oxygène s'écarte de la deuxième valeur d'oxygène de plus d'une valeur seuil d'oxygène prédéterminée,
- le fait de déterminer une valeur d'ammoniac sur la base d'une tension (V3) de potentiel mixte formée entre l'électrode (23) de potentiel mixte et l'électrode de référence (52), et
- le fait de détecter un échappement d'ammoniac dans la ligne d'échappement du moteur à combustion interne lorsque la valeur d'ammoniac détectée est supérieure à une valeur seuil d'ammoniac prédéterminée.

2. Procédé selon la revendication 1, dans lequel une défaillance de l'électrode (23) à potentiel mixte est déterminée lorsque la première valeur d'oxygène s'écarte de la deuxième valeur d'oxygène de plus que la valeur seuil d'oxygène prédéterminée.

3. Procédé selon l'une des revendications précédentes, dans lequel la valeur seuil d'ammoniac prédéterminée est d'environ 1 ppm.

4. Procédé selon l'une des revendications précédentes, comprenant en outre :
- le fait de constater qu'une détermination d'une défaillance du capteur (10) de gaz d'échappement n'est pas possible lorsque la première valeur d'oxygène détectée s'écarte d'une valeur de référence d'oxygène de plus d'une valeur seuil d'oxygène prédéterminée.

5. Procédé selon la revendication 4, dans lequel la valeur de référence d'oxygène se situe dans une plage allant d'environ 20 % à environ 21 %.

6. Procédé selon l'une des revendications précédentes, dans lequel l'état de fonctionnement prédéterminé du moteur à combustion interne comprend une poussée ou une décélération.

7. Procédé selon l'une des revendications précédentes, dans lequel la valeur seuil d'oxygène prédéterminée est d'environ 2 %, de préférence d'environ 1,5 % et, de manière particulièrement préférée, d'environ 1 %.

8. Capteur (10) de gaz d'échappement destiné à être agencé dans une ligne d'échappement d'un moteur à combustion interne, le capteur (10) de gaz d'échappement comprenant :
- un corps principal,
- une cavité de pompage (20) ménagée dans le corps principal (12) et reliée aux gaz d'échappement, dans laquelle est agencée une électrode de pompage (24),
- une électrode (23) à potentiel mixte, agencée à l'extérieur du corps principal (12) et en contact avec les gaz d'échappement,
- une cavité de référence (50), ménagée dans le corps principal (12) et reliée à l'air ambiant, dans laquelle est agencée une électrode de référence (52), et
- une unité de commande qui est conçue pour mettre en œuvre un procédé selon l'une des revendications précédentes.
